# EUROPEAN PATENT APPLICATION

(11) **EP 4 209 268 A1**
(43) Date of publication of application: **12.07.2023**
(21) Application number: 21864168.6
(22) Date of filing: 23.08.2021
(51) Int. Cl.: B01J 23/888, B01J 37/04, B01J 37/08, C07C 51/235, C07C 57/055

(54) **CATALYST FOR ACRYLIC ACID PRODUCTION, METHOD FOR PRODUCING SAME, AND METHOD FOR PRODUCING ACRYLIC ACID**

(30) Priority: 03.09.2020 JP 2020148124
(71) Applicant: Nippon Shokubai Co., Ltd., Osaka-shi, Osaka 541-0043 (JP)
(72) Inventor: IGUCHI, Norihiko, Himeji-shi, Hyogo 671-1282 (JP)
(74) Representative: Müller-Boré & Partner Patentanwälte PartG mbB
(86) International application number: PCT/JP2021/030816
(87) International publication number: WO 2022/050110

(57) **Abstract**

A catalyst for producing acrylic acid from acrolein, comprising a catalytic active component containing molybdenum, vanadium and copper, wherein: the catalytic active component has diffraction peaks at 2θ = 22.2° ± 0.3°, 28.2° ± 0.3°, 31.5° ± 0.3°, and 32.6° ± 0.3° in X-ray diffraction analysis using Cu-Kα radiation; a ratio I(28.2°)/I(22.2°) of peak intensity at 2θ = 28.2° ± 0.3° and peak intensity at 2θ = 22.2° ± 0.3° is 0.20 or more and 0.50 or less; a ratio I(31.5°)II(22.2°) of peak intensity at 2θ = 31.5° ± 0.3° and the peak intensity at 2θ = 22.2° ± 0.3° is 0.03 or more and 0.20 or less; and a ratio I(32.6°)/I(22.2°) of peak intensity at 2θ = 32.6° ± 0.3° and the peak intensity at 2θ = 22.2° ± 0.3° is 0.03 or more and 0.40 or less.

## Description

### TECHNICAL FIELD

The present invention relates to a catalyst for producing acrylic acid from acrolein, a method for preparing the same, and a process for producing acrylic acid using the catalyst.

### BACKGROUND ART

Acrylic acid is widely used as a raw material for various industrial products. Currently, a general method for producing acrylic acid is a method of gas-phase oxidation of propylene in the presence of a catalyst using a fixed bed reactor, and according to this production method, propylene is subjected to gas-phase catalytic oxidation in a first-stage reaction to obtain an acrolein-containing gas, and is further subjected to gas-phase catalytic oxidation in a second-stage reaction to obtain acrylic acid. As the catalyst used here, catalysts containing molybdenum as an essential component is generally used. Among them, catalysts comprising a catalytic active component containing molybdenum and vanadium, that is, catalysts containing a Mo-V composite oxide, are known as a catalyst used in the second-stage reaction for producing acrylic acid from acrolein. In general, performance of a catalyst is known to depend greatly not only on a kind and content of metals constituting the catalytic active component, but also on a crystal structure of the catalytic active component, and for example, Patent Literatures 1 to 5 disclose catalysts having crystal structures that exhibit various X-ray diffraction profiles in X-ray diffraction analysis.

It is also known that the crystal structure of the catalytic active component generally changes greatly depending on a method for preparing the catalyst and its preparing conditions. For example, the above Patent Literatures 1 to 5 discloses: a method in which mixed solution of raw material compounds is dried and then calcined to coat a carrier (Patent Literature 1); a method of blowing an oxygen-containing gas in a step of reacting raw material compounds in an aqueous medium (Patent Literature 2); a method in which a nitrogen-containing compound which is different from starting materials is added in any of the step of obtaining a mixed solution of the starting materials, the step of drying the mixed solution to obtain a dried product or further calcining the dried product to obtain a calcined product, and the step of molding the dried or calcined product or supporting it on an inert carrier (Patent Literature 3); a method in which an additive which is different from starting materials is added in any of the step of obtaining a mixed solution of the starting materials, the step of drying the mixed solution to obtain a dried product, and the step of supporting the dried product on an inert carrier (Patent Literature 4); and a method of separately preparing a mixed solution containing molybdenum and vanadium and a mixed solution of an antimony compound and a basic aqueous solution, and then mixing the two solutions (Patent Literature 5), and catalysts having crystal structures that exhibit various X-ray diffraction profiles are respectively obtained, as described above.

The crystal structure of the catalytic active component is considered to be affected by conditions in a calcination step in preparation, and in the above Patent Literatures 1 to 5, the temperature is generally adjusted appropriately within a temperature range of 250°C to 660°C. Meanwhile, Patent Literature 6 discloses a method for preparing a catalytic active multi-element oxide material containing Mo, V and Cu, wherein a homogeneous dry mixture containing starting materials is prepared, which is calcined at a temperature of 380°C to 450°C finally, and then the homogeneous dry mixture is cooled to a temperature of 100°C or lower taking within 5 hours.

### CITATION LIST

### PATENT LITERATURE

PATENT LITERATURE 1
   Japanese Unexamined Patent Application Publication No. H8-299797
PATENT LITERATURE 2
   Japanese Unexamined Patent Application Publication No. H11-285637
PATENT LITERATURE 3
   Japanese Unexamined Patent Application Publication No. 2015-120133
PATENT LITERATURE 4
   Japanese Unexamined Patent Application Publication No. 2016-59898
PATENT LITERATURE 5
   Japanese Unexamined Patent Application Publication No. 2018-43197
PATENT LITERATURE 6
   Japanese Unexamined Patent Application Publication No. 2006-526495

### SUMMARY OF INVENTION

### TECHNICAL PROBLEM

The catalysts disclosed in the above documents having various crystal structures exhibit high performance in some extent as practical catalysts for producing acrylic acid from acrolein; however, there is still room for improvement, and further performance improvement, specifically, higher selectivity or higher yield is required. The present invention has been made in view of the above circumstances, and an object the present invention is to provide a catalyst for producing acrylic acid from acrolein, that exhibits high acrylic acid selectivity or high acrylic acid yield, and to provide a preparation method for easily obtaining a catalyst that exhibits high acrylic acid selectivity or high acrylic acid yield. The present invention also provides a process for producing acrylic acid from acrolein using the catalyst of the present invention or the catalyst obtained by the preparation method of the present invention.

### SOLUTION TO PROBLEM

In order to achieve the above object, the present inventor has conducted various studies, focusing on optimizing a crystal structure of a catalytic active component, and as a method for obtaining a catalytic active component having an optimal crystal structure, focusing on cooling conditions following a calcination step. As a result, the present inventor has found that catalysts comprising a catalytic active component which has diffraction peaks at specific diffraction angles in X-ray diffraction analysis and in which each of the peak intensity ratios shows a value within a certain range exhibit high acrylic acid selectivity or high acrylic acid yield when producing acrylic acid by gas-phase oxidation of acrolein, and that it is effective to appropriately set a cooling period and a gas atmosphere for obtaining catalysts comprising such a catalytic active component, and have completed the present invention.

Thus, the present invention includes the following inventions.
[1] A catalyst for producing acrylic acid from acrolein, comprising a catalytic active component containing molybdenum, vanadium and copper, wherein: the catalytic active component has diffraction peaks at 2θ = 22.2° ± 0.3°, 28.2° ± 0.3°, 31.5° ± 0.3°, and 32.6° ± 0.3° in X-ray diffraction analysis using Cu-Kα radiation; a ratio I(28.2°)/I(22.2°) of peak intensity at 2θ = 28.2° ± 0.3° and peak intensity at 2θ = 22.2° ± 0.3° is 0.20 or more and 0.50 or less; a ratio I(31.5°)/I(22.2°) of peak intensity at 2θ = 31.5° ± 0.3° and the peak intensity at 2θ = 22.2° ± 0.3° is 0.03 or more and 0.20 or less; and a ratio I(32.6°)/I(22.2°) of peak intensity at 2θ = 32.6° ± 0.3° and the peak intensity at 2θ = 22.2° ± 0.3° is 0.03 or more and 0.40 or less.
[2] The catalyst for producing acrylic acid according to [1], wherein: the catalytic active component further has a diffraction peak at 2θ = 33.4° ± 0.3° in the X-ray diffraction analysis; a ratio I(33.4°)/I(22.2°) of peak intensity at 2θ = 33.4° ± 0.3° and the peak intensity at 2θ = 22.2° ± 0.3° is 0.06 or more and 0.40 or less; and a ratio I(5°-10°)/I(22.2°) of the highest intensity in a range of 2θ = 5° to 10° and the peak intensity at 2θ = 22.2° ± 0.3° is 0.06 or less.
[3] The catalyst for producing acrylic acid according to [1] or [2], wherein: the catalytic active component further has a diffraction peak at 2θ = 27.3° ± 0.3° in the X-ray diffraction analysis; and a ratio I(27.3°)/I(22.2°) of peak intensity at 2θ = 27.3° ± 0.3° and the peak intensity at 2θ = 22.2° ± 0.3° is 0.10 or more and 0.60 or less.
[4] The catalyst for producing acrylic acid according to any one of [1] to [3], wherein: the catalytic active component has a composition represented by the following formula (1): MoₐV_{b}Cu_{c}A_{d}BₑC_{f}D_{g}EₕOᵢ ···(1), wherein Mo is molybdenum, V is vanadium, Cu is copper, A represents at least one element selected from the group consisting of tungsten, niobium and tantalum, B represents at least one element selected from the group consisting of antimony and tin, C represents at least one element selected from the group consisting of chromium, manganese, iron, cobalt, nickel, zinc, zirconium, tellurium, cerium and bismuth, D represents at least one element selected from the group consisting of alkali metals and alkaline earth metals, E represents at least one element selected from the group consisting of silicon, aluminum and titanium, O is oxygen, a to i mean atomic ratios of Mo, V, Cu, A, B, C, D, E and O, respectively, and meet inequalities: a=12, 2≤b≤14, 0<c≤5, 0<d≤10, 0<e≤5, 0≤f≤5, 0≤5g≤3, 0≤h≤30, respectively, and i is a numerical value determined by oxidation states of respective elements.
[5] A method for preparing a catalyst for producing acrylic acid from acrolein, comprising the steps of: obtaining a raw material mixed solution containing molybdenum, vanadium and copper; drying the raw material mixed solution to obtain a dried product; molding the dried product or supporting the dried product on an inert carrier to obtain a molded product or a supported product; calcining the molded product or the supported product at a temperature Tc of 380°C or higher and 460°C or lower; and cooling from the temperature Tc to 100°C over 5 hours or longer, after the calcining, in an atmosphere with an oxygen concentration of more than 5 volume% and 21 volume% or less.
[6] A method for preparing a catalyst for producing acrylic acid from acrolein, comprising the steps of: obtaining a raw material mixed solution containing molybdenum, vanadium and copper; drying the raw material mixed solution to obtain a dried product; calcining the dried product at a temperature Tc of 380°C or higher and 460°C or lower; cooling from the temperature Tc to 100°C over 5 hours or longer, after the calcining, in an atmosphere with an oxygen concentration of more than 5 volume% and 21 volume% or less; and molding a calcined product obtained through the cooling step or supporting a calcined product obtained through the cooling step on an inert carrier.
[7] The method for preparing a catalyst for producing acrylic acid according to [5] or [6], wherein cooling from the temperature Tc to 300°C over 3 hours or longer in the cooling step.
[8] A process for producing acrylic acid comprising the step of conducing gas-phase catalytic oxidation of acrolein in the presence of the catalyst according to any one of [1] to [4] or the catalyst obtained by the preparing method according to any one of [5] to [7].

### ADVANTAGEOUS EFFECTS OF INVENTION

The catalyst for producing acrylic acid of the present invention exhibits high acrylic acid selectivity or high acrylic acid yield. Further, according to the method for preparing a catalyst for producing acrylic acid of the present invention, the catalyst for producing acrylic acid of the present invention can be easily obtained, and the obtained catalyst exhibits high acrylic acid selectivity or high acrylic acid yield.

### DESCRIPTION OF THE PREFERRED EMBODIMENTS

The present invention relates to a catalyst for producing acrylic acid and a method for producing the same, and more particularly relates to a catalyst for producing acrylic acid by gas-phase catalytic oxidation reaction of acrolein and a method for preparing the same. The catalyst of the present invention comprises a catalytic active component containing molybdenum, vanadium and copper, and specifically, it contains a composite oxide containing the elements of molybdenum, vanadium and copper. In the present invention, a composite oxide containing the elements of molybdenum, vanadium and copper can be used as a catalyst. Hereinafter, a composite oxide containing the elements of molybdenum, vanadium and copper is referred to as a "Mo-V-Cu composite oxide"; however, this description does not exclude the inclusion of elements other than Mo, V and Cu. Mo-V composite oxide catalysts are widely used industrially as a catalyst for oxidizing acrolein to produce acrylic acid, and the catalyst of the present invention comprises a Mo-V-Cu composite oxide, that further contains Cu in addition to Mo and V. By using a Mo-V-Cu composite oxide, which contains Cu, acrylic acid selectivity can be increased.

In the catalyst of the present invention, the catalytic active component may contain another metal or semi-metal element in addition to molybdenum, vanadium and copper. Examples of another metal or semi-metal element includes, for example, tungsten, niobium, tantalum, antimony, tin, chromium, manganese, iron, cobalt, nickel, zinc, zirconium, tellurium, cerium, bismuth, alkali metals, alkaline earth metals, silicon, aluminum, titanium and others, and the Mo-V-Cu composite oxide of the catalytic active component preferably further contains at least one element selected from these elements. Among them, in the catalyst of the present invention, it is preferable that the catalytic active component further contains tungsten and/or antimony in addition to molybdenum, vanadium and copper, and thus, it is preferable that the Mo-V-Cu composite oxide further contains tungsten and/or antimony.

In the catalyst of the present invention, the catalytic active component preferably has a composition represented by, for example, the following formula (1):

MoₐV_{b}Cu_{c}A_{d}BₑC_{f}D_{g}EₕOᵢ ... (1)

In the above formula (1), Mo is molybdenum, V is vanadium, Cu is copper, A represents at least one element selected from the group consisting of tungsten, niobium and tantalum, B represents at least one element selected from the group consisting of antimony and tin, C represents at least one element selected from the group consisting of chromium, manganese, iron, cobalt, nickel, zinc, zirconium, tellurium, cerium and bismuth, D represents at least one element selected from the group consisting of alkali metals and alkaline earth metals, E represents at least one element selected from the group consisting of silicon, aluminum and titanium, O is oxygen, a to i mean atomic ratios of Mo, V, Cu, A, B, C, D, E and O, respectively, and meet inequalities: a=12, 2≤b≤14, 0<c≤5, 0<d≤10, 0<e≤5, 0≤f≤5, 0≤g≤3 , 0≤h≤30, respectively, and i is a numerical value determined by oxidation states of respective elements. In the above formula (1), provided a=12, the value of b is preferably 3 or more, and preferably 10 or less, more preferably 8 or less. The value of c is preferably 0.1 or more, more preferably 0.2 or more, and preferably 4 or less. The value of d is preferably 0.1 or more, more preferably 0.3 or more, even more preferably 0.5 or more, and preferably 6 or less, more preferably 4 or less, even more preferably 3 or less. The value of e is preferably 0.1 or more, more preferably 0.2 or more, and preferably 3 or less, more preferably 2 or less, even more preferably 1 or less. The value of f is preferably 4 or less, more preferably 3 or less, and may be 0. The value of g is preferably 2 or less, more preferably 1 or less, and may be 0. The value of h is preferably 20 or less, more preferably 10 or less, and may be 0.

In the above formula (1), A is preferably tungsten and B is preferably antimony. That is, in the catalyst of the present invention, the catalytic active component preferably further contains tungsten and antimony in addition to molybdenum, vanadium and copper.

In the above formula (1), the values of f, g and h may be 0. In this case, the catalyst of the present invention preferably comprises the catalytic active component having a composition represented by the following formula (2). In the following formula (2), Mo, V, Cu, A, B, O, a-e, and i have the same meanings as above.

MoₐV_{b}Cu_{c}A_{d}BₑOᵢ ··· (2)

In the above formula (2), A is preferably tungsten and B is preferably antimony, and therefore, in the catalyst of the present invention, it is more preferable that the catalytic active component has a composition represented by the following formula (3). In the following formula (3), Mo, V, Cu, O, a-e, and i have the same meanings as above, W is tungsten and Sb is antimony.

MoₐV_{b}Cu_{c}W_{d}SbₑOᵢ ... (3)

The composition of the catalytic active component, namely, a catalyst composition, can be determined by measuring metal components contained in the catalyst using an ICP emission spectrometer, an atomic absorption spectrometer, or a fluorescent X-ray spectrometer. Or, the catalyst composition may be determined from the amount of metal components used as raw materials in preparing the catalyst and/or the amount of metal components actually taken into the catalyst.

In X-ray diffraction analysis using Cu-Kα radiation, the catalytic active component has diffraction peaks at 2θ = 22.2° ± 0.3°, 28.2° ± 0.3°, 31.5° ± 0.3°, and 32.6°±0.3°. The catalyst of the present invention is different from conventionally known Mo-V composite oxide catalysts for producing acrylic acid in that it exhibits certain peak intensities at these diffraction angles. Specifically, a ratio I(28.2°)/I(22.2°) of peak intensity at 2θ = 28.2° ± 0.3° and peak intensity at 2θ = 22.2° ± 0.3° is 0.20 or more and 0.50 or less, a ratio I(31.5°)/I(22.2°) of peak intensity at 2θ = 31.5° ± 0.3° and the peak intensity at 2θ = 22.2° ± 0.3° is 0.03 or more and 0.20 or less, and a ratio I(32.6°)/I(22.2°) of peak intensity at 2θ = 32.6° ± 0.3° and the peak intensity at 2θ = 22.2° ± 0.3° is 0.03 or more and 0.40 or less. The ratio I(28.2°)/I(22.2°) is preferably 0.22 or more, and more preferably 0.23 or more. The ratio I(31.5°)II(22.2°) is preferably 0.18 or less, and more preferably 0.16 or less. The ratio I(32.6°)/I(22.2°) is preferably 0.38 or less, and more preferably 0.35 or less.

Since the catalytic active component has diffraction peaks at the specific diffraction angles in X-ray diffraction analysis as described above, and each of the above peak intensity ratios has a value within a certain range, the catalyst of the present invention exhibits high acrylic acid selectivity or high acrylic acid yield when producing acrylic acid by gas-phase oxidation of acrolein. Explaining this in detail, the diffraction peak at 2θ = 22.2° ± 0.3° can be assigned to VMo₃O₁₁, that is conventionally known to be an effective structure for the reaction from acrolein to acrylic acid. The diffraction peak at 2θ = 28.2° ± 0.3° can be assigned mainly to VMo₃O₁₁ (however, the crystal plane is different from that in the diffraction peak at 2θ = 22.2° ± 0.3°), and the diffraction peaks at 2θ = 31.5° ± 0.3° and 2θ = 32.6° ± 0.3° can be mainly assigned to (V_{0.07}Mo_{0.93})₅O₁₄ and V_{0.95}Mo_{0.97}O₅, respectively, of which crystal structures themselves are different from that of the diffraction peak at 2θ = 22.2° ± 0.3°. As a result of studies by the present inventor, it has been found that when the crystal plane and the crystal structure which are different from the structure assigned to the diffraction peak at 2θ = 22.2° ± 0.3° exist in an excessively large amount or in an excessively small amount in the catalytic active component, the catalyst becomes less active and/or less selective to acrylic acid in the reaction from acrolein to acrylic acid. However, in the catalysis of the present invention, since a plurality of crystal planes and crystal structures which are different from the structure assigned to the diffraction peak at 2θ = 22.2° ± 0.3° exist in the catalytic active component in a certain range of the ratios to the structure assigned to the diffraction peak at 2θ = 22.2° ± 0.3°, it is presumed that oxygen transfer ability or adsorption/desorption ability of acrolein, acrylic acid or the like in the gas-phase catalytic oxidation reaction of acrolein to acrylic acid is improved, whereby performance of the catalytic activity or selectivity is improved. The above assignments of the diffraction angles are based on the powder diffraction and crystal structure database (ICDD).

It is preferable that the catalyst of the present invention further has a diffraction peak at 2θ = 33.4° ± 0.3° with a certain intensity in the X-ray diffraction analysis of the catalytic active component using Cu-Kα radiation. Specifically, it is preferable that a ratio I(33.4°)/I(22.2°) of peak intensity at 2θ = 33.4° ± 0.3° and the peak intensity at 2θ = 22.2° ± 0.3° is 0.06 or more and 0.40 or less. The ratio I(33.4°)/I(22.2°) is more preferably 0.38 or less, and even more preferably 0.35 or less. It is also preferable that, in the X-ray diffraction analysis of the catalytic active component using Cu-Kα radiation, the highest intensity in the range of 2θ = 5° to 10° is less than a certain value compared to the peak intensity at 2θ = 22.2° ± 0.3°. Specifically, a ratio I(5°-10°)/I(22.2°) of the highest intensity in the range of 2θ = 5° to 10° and the peak intensity at 2θ = 22.2° ± 0.3° is preferably 0.06 or less, and more preferably 0.05 or less. Here, "the highest intensity in the range of 2θ = 5° to 10°" means the highest intensity value in that range, in spite that a clear peak appears or no clear peak present in that range of the diffraction angle. In the catalyst of the present invention, when the catalytic active component has such an X-ray diffraction profile, high acrylic acid selectivity or high acrylic acid yield is exhibited more reliably in producing acrylic acid by gas-phase catalytic oxidation of acrolein.

The catalytic active component may further have a diffraction peak at 2θ = 27.3° ± 0.3° in the X-ray diffraction analysis of the catalytic active component using Cu-Kα radiation. For example, a ratio I(27.3°)/I(22.2°) of peak intensity at 2θ = 27.3° ± 0.3° and the peak intensity at 2θ = 22.2° ± 0.3° may be 0.10 or more and 0.60 or less. The ratio I(27.3°)/I(22.2°) may be 0.14 or more, 0.16 or more, or 0.20 or more, and may be 0.50 or less, or 0.40 or less. In the catalyst of the present invention, when the catalytic active component has such an X-ray diffraction profile, high acrylic acid selectivity or high acrylic acid yield is exhibited more reliably in producing acrylic acid by gas-phase catalytic oxidation of acrolein.

A shape of the catalyst is not particularly limited, and examples thereof include granular shape, spherical shape, columnar shape, ring shape, saddle shape, honeycomb shape, irregular shape and others. A particle size of the catalyst is preferably 1.0 mm or larger, more preferably 2.0 mm or larger, even more preferably 2.5 mm or larger, and preferably 30.0 mm or smaller, more preferably 20.0 mm or smaller, even more preferably 15.0 mm or smaller.

The catalyst of the present invention may be a supported catalyst in which the catalytic active component containing a Mo-V-Cu composite oxide is supported on a carrier, or may be a non-supported catalyst in which the catalytic active component is not supported on a carrier. The carrier is preferably inert to acrolein and acrylic acid, and inorganic oxides or composite oxides such as silica, alumina, titania and zirconias; crystalline metallosilicates such as zeolite; metals or alloys such as stainless steels and aluminum; inorganic carbon compounds such as activated carbon and silicon carbide; or the like can be used. Among them, silica, alumina, titania, zirconia, or composite oxides thereof is preferably used as the carrier.

A particle size of the carrier is not particularly limited, and in the case where the catalyst is filled in a reaction tube of a (multi)tubular reactor, the carrier may have a desired particle size as described above, which can be filled into the reaction tube when it is made into a supported catalyst, and thus, a particle size of the supported catalyst may be appropriately set to be less than an inner diameter of the reaction tube.

A supporting rate of the supported catalyst may be adjusted as appropriate from the viewpoint of facilitating an operation control in the reactor and/or controlling a life of the catalyst, and for example, it is preferably 10 mass% or more, more preferably 20 mass% or more, and preferably 300 mass% or less, more preferably 200 mass% or less. The supporting rate of the supported catalyst can be determined from the following formula: supporting rate (%) of the supported catalyst = mass of the supported catalytic active component / mass of the carrier × 100.

Next, a method for preparing a catalyst according to the present invention is explained. The catalyst of the present invention is preferably produced by the following-described method for preparing a catalyst for producing acrylic acid according to the present invention. The catalyst of the present invention can be easily prepared by the following preparing method. The catalyst of the present invention may be obtained by the method for preparing a catalyst for producing acrylic acid according to the present invention described below.

The method for preparing a catalyst according to the present invention is a method for preparing a catalyst for producing acrylic acid from acrolein, comprising steps of: obtaining a raw material mixed solution containing molybdenum, vanadium and copper; drying the raw material mixed solution to obtain a dried product; molding the dried product or supporting the dried product on an inert carrier to obtain a molded product or a supported product; calcining the molded product or the supported product at a temperature Tc of 380°C or higher and 460°C or lower; and cooling from the temperature Tc to 100°C over 5 hours or longer, after the calcinating, in an atmosphere with an oxygen concentration of more than 5 volume% and 21 volume% or less (hereinafter referred to as a "first preparing method"). Also, the method for preparing a catalyst according to the present invention is a method for preparing a catalyst for producing acrylic acid from acrolein, comprising steps of: obtaining a raw material mixed solution containing molybdenum, vanadium and copper; drying the raw material mixed solution to obtain a dried product; calcining the dried product at a temperature Tc of 380°C or higher and 460°C or lower; cooling from the temperature Tc to 100°C over 5 hours or longer, after the calcinating, in an atmosphere with an oxygen concentration of more than 5 volume% and 21 volume% or less; and molding a calcined product obtained through the cooling step or supporting a calcined product obtained through the cooling step on an inert carrier (hereinafter referred to as a "second preparing method"). The methods for preparing a catalyst according to the present invention are described in detail below.

In the step of obtaining a raw material mixed solution, a raw material mixed solution containing molybdenum, vanadium and copper is prepared as raw materials for a catalytic active component. As described above, in the case where the catalytic active component contains another metal or semi-metal element in addition to molybdenum, vanadium and copper, it is preferable that the raw material mixed solution further contains these metal or semi-metal elements.

The raw material mixed solution can be prepared by dissolving or dispersing a molybdenum compound, a vanadium compound and a copper compound as raw material compounds in a solvent or a dispersion medium. The solvent or dispersion medium for the raw material mixed solution is not particularly limited; however, it is convenient to use water. Therefore, the raw material mixed solution is preferably obtained as an aqueous solution of a molybdenum compound, a vanadium compound and a copper compound dissolved in water or as a dispersion (slurry) thereof in water, and the aqueous solution or dispersion may further contain another metal compound or semi-metal compound dissolved or dispersed in it.

The forms of the molybdenum compound, the vanadium compound and the copper compound are not particularly limited, and any form of compound such as: oxides; hydroxides; halides such as chlorides, bromides and iodides; inorganic salts such as nitrates, carbonates and sulfates; organic acid salts such as formate, acetates, oxalates and citrate; oxoacids or oxoacid salts (e.g., ammonium salts or alkali metal salts of oxoacids) can be used. Among them, as the molybdenum compound, oxoacids or oxoacid salts are preferable, and paramolybdic acid or ammonium paramolybdate is more preferable. As the vanadium compound, oxoacids or oxoacid salts are preferable, and metavanadic acid or ammonium metavanadate is more preferable. As the copper compound, inorganic salts are preferable, and copper nitrate is more preferable. In the case where the catalytic active component contains another metal or semi-metal element in addition to molybdenum, vanadium and copper, these metal or semi-metal elements can also be used in any form of compound. Each of these compounds may be selected as appropriate in consideration of solubility in a solvent or the like. The raw material mixed solution may be appropriately stirred and/or heated to increase the solubility.

The amount of the molybdenum compound, the vanadium compound and the copper compound to be used may be appropriately set according to the desired composition of the catalytic active component. For example, a molar ratio Mo/V of molybdenum (Mo) and vanadium (V) in the raw material mixed solution is preferably 0.8 or more, more preferably 1.2 or more, even more preferably 1.5 or more, and preferably 6.0 or less, more preferably 4.0 or less. Further, a molar ratio Mo/Cu of molybdenum (Mo) and copper (Cu) in the raw material mixed solution is preferably 2.4 or more, more preferably 3.0 or more, and preferably 240 or less, more preferably 120 or less, even more preferably 60 or less.

In the case where the catalytic active component contains another metal or semi-metal element in addition to molybdenum, vanadium and copper, the amount of the compound of the metal or semi-metallic element to be used may also be appropriately set according to the desired catalytic active component. In the present invention, it is preferable that the catalytic active component further contains tungsten and/or antimony. For example, in the case where the catalytic active component further contains tungsten, a molar ratio Mo/W of molybdenum (Mo) and tungsten (W) is preferably 1.2 or more, more preferably 2.0 or more, even more preferably 4.0 or more, still even more preferably 6.0 or more, and preferably 240 or less, more preferably 120 or less, even more preferably 40 or less, still even more preferably 24 or less. In the case where the catalytic active component further contains antimony, a molar ratio Mo/Sb of molybdenum (Mo) and antimony (Sb) is preferably 2.4 or more, more preferably 3.0 or more, even more preferably 4.0 or more, and preferably 240 or less, more preferably 120 or less, even more preferably 60 or less.

In the case where the catalytic active component further contains tungsten and/or antimony, the forms of a tungsten compound and an antimony compound as raw material compounds are not particularly limited, and any form of compound above described can be used. Among them, as the tungsten compound, oxoacids or oxoacid salts is preferable, and paratungstic acid or ammonium paratungstate is more preferable. As the antimony compound, antimony trioxide is preferable.

In the step of obtaining the raw material mixed solution, the molybdenum compound, the vanadium compound and the copper compound may be dissolved or dispersed all at once to prepare the raw material mixed solution, or solutions or dispersions may be prepared for each compound and mixed to prepare the raw material mixed solution. Alternatively, a single or mixed solution or a single or mixed dispersion of one or two compounds selected from the molybdenum compound, the vanadium compound and the copper compound may be prepared, a single or mixed solution or a single or mixed dispersion of the remaining one or two compounds may be prepared, and they may be mixed to prepare the raw material mixed solution. Also in the case where the catalytic active component contains another metal or semi-metal element in addition to molybdenum, vanadium and copper, the method of dissolving and mixing each compound is not particularly limited, and it is sufficient to obtain the raw material mixed solution in which all or part of the raw material compounds are finally dissolved.

The raw material mixed solution obtained in the step of obtaining the raw material mixed solution may be subjected to a subsequent drying step immediately after preparation, or the drying step may be performed after continuing stirring. Stirring of the raw material mixed solution may be accompanied by heating. The temperature at which the raw material mixed solution is stirred is not particularly limited, and is preferably 20°C or higher, more preferably 25°C or higher, even more preferably 30°C or higher, and preferably 100°C or lower, more preferably 90°C or lower. A stirring period for continuously stirring the raw material mixed solution is not particularly limited, and may be 1 hour or longer, 5 hours or longer, or 10 hours or longer, and may be 360 hours or shorter, 240 hours or shorter, or 120 hours or shorter.

Following the step of obtaining the raw material mixed solution, the drying step of drying the raw material mixed solution is conducted. By drying the raw material mixed solution, a dried product is obtained. As a drying method, a known method such as heating or pressure reduction may be adopted. For example, a powdery dried product may be obtained by heating the raw material mixed solution using a spray dryer, a drum dryer or the like, or a block-shaped or flake-shaped dried product may be obtained by heating the raw material mixed solution under gas-flowing or without gas-flowing using a box-type dryer, a tunnel-type dryer or the like. Examples of a gas introduced for gas-flowing or an atmospheric gas without gas-flowing include an inert gas such as nitrogen, an oxygen-containing gas such as air, and others. Or, once the raw material mixed solution is concentrated and/or evaporated to dryness to obtain a cake-like solid matter, the solid matter may be further heated to obtain the dried product. In the case where the drying is performed by heating, the drying treatment is preferably conducted at a temperature of, for example, 60°C to 180°C. A drying period is not particularly limited; however, it is preferably 1 hour or longer, more preferably 2 hours or longer, and preferably 48 hours or shorter, more preferably 24 hours or shorter, from the viewpoint of production efficiency of the catalyst. In the case where the drying is performed under reduced pressure, the raw material mixed solution may be dried under reduced pressure using a vacuum dryer or the like to obtain a block-shaped or powdery dried product.

The obtained dried product may be pulverized and/or classified as necessary to adjust to an appropriate particle size. The particle size is not particularly limited; however, a particle size in which the particle fraction passing through a sieve with an opening of 500 µm is 90 mass% or more is preferable, and a particle size in which the particle fraction passing through a sieve with an opening of 300 µm is 90 mass% or more is more preferable, for example. The dried product which has been pulverized and/or classified is also included in the dried product in the present invention.

In the first preparing method, then, a step of molding the dried product obtained in the drying step or supporting it on an inert carrier to obtain a molded product or a supported product is conducted (hereinafter referred to as a "first molding/supporting step"). In the case of molding the dried product, the dried product may be molded into a certain shape by a known molding method such as an extrusion molding method or a tableting molding method. A shape of the molded product is not particularly limited, and examples thereof include spherical shape, columnar shape, ring shape, saddle shape, irregular shape and others. In the case of supporting the dried product on an inert carrier, the dried product may be supported on any inert carrier having a certain shape to obtain the supported product. The material of the inert carrier used for supporting the dried product is as explained above. A shape of the inert carrier is not particularly limited, and examples thereof include granular shape, spherical shape, columnar shape, ring shape, saddle shape, honeycomb shape, irregular shape and others.

When molding or supporting the dried product, substances such as molding aids and binders for improving moldability and supportability of the dried product, and pore-forming agents for forming appropriate pores in the catalyst, those are generally used for these effects in production of catalysts (that may be referred to as an "auxiliary substances"), can be used, and among them, it is preferable to use a binder. Specific examples of the binder include organic compounds such as ethylene glycol, glycerin, propionic acid, maleic acid, benzyl alcohol, propyl alcohol, butyl alcohol, phenols and celluloses (e.g., cellulose and ethyl cellulose), water, nitric acid, ammonium nitrate, ammonium carbonate, and others. These may be used alone or in combination of two or more. In addition to the auxiliary substance, a reinforcing agent can also be used for the purpose of improving mechanical strength of the catalyst. Specific examples of reinforcing agents include various whiskers such as silica, alumina, ceramic fibers, glass fibers, carbon fibers, mineral fibers, metal fibers, silicon carbide and silicon nitride, and the crystalline structure thereof may be polycrystalline, monocrystalline or amorphous, and polycrystalline or monocrystalline is preferable. Moreover, a plurality of reinforcing agents having different fiber diameters, fiber lengths, or made of different materials may be used depending on the shape of the catalyst or the desired mechanical strength. The reinforcing agent may be added to the raw material mixed solution, or may be mixed with the dried product when molding or supporting.

There are no particular restrictions on equipment used to support the dried product on the inert carrier, and any conventionally known mixing equipment can be used. For example, it is preferable to support the dried product on the inert carrier using by a centrifugal fluid coating method described in Japanese Unexamined Patent Application Publication No. S63-200839 or a rocking mixer method described in Japanese Unexamined Patent Application Publication No. 2004-136267.

In the first preparing method, next, a calcining step of calcining the molded product or the supported product obtained in the first molding/supporting step at a temperature Tc of 380°C or higher and 460°C or lower is conducted. The calcining temperature Tc in the calcining step is preferably 390°C or higher and preferably 450°C or lower. In the calcining step, a rate of temperature increase to raise a set temperature is preferably 0.1°C/min or more, more preferably 0.5°C/min or more, and preferably 15°C/min or less, more preferably 10°C/min or less. A calcining period at the calcining temperature Tc may be appropriately adjusted between, for example, 0.5 hours and 12 hours. An atmosphere for calcining is not particularly limited, and can be performed in an inert gas atmosphere such as nitrogen or argon, a reducing gas atmosphere such as hydrogen, or an oxygen-containing gas atmosphere such as air. Among them, it is preferable to be performed in an oxygen-containing gas atmosphere. The oxygen concentration at that time is preferably 1 volume% or more, and preferably 21 volume% or less.

In the second preparing method, a calcining step of calcining the dried product which has been dried in the drying step or further pulverized and/or classified at a temperature Tc of 380°C or higher and 460°C or lower is conducted. Explanations of the calcining step in the second preparing method is referred to the description of the calcining step in the first preparing method.

A calcining furnace used in the calcining step of the first or second preparing method is not particularly limited, and a generally used box-type calcining furnace, tunnel-type calcining furnace, or the like can be used. A method for measuring the calcining temperature is described later.

In the first or second preparing method, a cooling step is performed after calcining in the calcining step. Specifically, the dried product, molded product or supported product after the calcining is cooled from the temperature Tc to 100°C over 5 hours or longer in an atmosphere with an oxygen concentration of more than 5 volume% and 21 volume% or less. By cooling in this manner, the catalyst of the present invention, that is, a catalyst having diffraction peaks at the specific diffraction angles and having certain peak intensity ratios in X-ray diffraction analysis of the catalytic active component as described above, can be easily obtained. Therefore, the catalyst obtained by the method for preparing a catalyst according to the present invention exhibits high acrylic acid selectivity or high acrylic acid yield when producing acrylic acid by gas-phase oxidation of acrolein. The cooling step is preferably carried out following the calcining step, and it is preferable that after calcining the dried product, molded product or supported product in a calcining furnace, cooling is successively conducted in that calcining furnace.

In the cooling step, the cooling period and the gas atmosphere in the furnace have a great influence on the crystal structure of the resulting catalytic active component. Explaining this in detail, crystal growth proceeds by solid-phase reaction during calcining in the calcining step, and since the temperature does not immediately drop even in the cooling step after calcining, the crystal growth further proceeds by continuing the solid-phase reaction. At this time, by controlling the cooling period and the oxygen concentration in the cooling step as described above, it is thought the crystal growth after calcining (in the cooling step) becomes moderate, so that the catalyst having diffraction peaks at the specific diffraction angles and having certain peak intensity ratios as described above, can be easily obtained. For example, in the cooling step, by cooling from the temperature Tc to 100°C over the cooling period of 5 hours or longer, the catalytic active component of the obtained catalyst comes to have the peak intensity ratio I(31.5°)/I(22.2°) of 0.03 or more in X-ray diffraction analysis and to exhibit high acrylic acid selectivity or high acrylic acid yield. By setting the oxygen concentration in the atmosphere in the cooling step to more than 5 volume%, the catalytic active component of the obtained catalyst comes to have the peak intensity ratio I(28.2°)/I(22.2°) of 0.50 or less and to exhibit high acrylic acid selectivity or high acrylic acid yield. By setting the oxygen concentration in the atmosphere in the cooling step to 21 volume% or less, the catalytic active component of the obtained catalyst comes to have the peak intensity ratio I(32.6°)/I(22.2°) of 0.40 or less. Since this catalyst has high activity and allows the reaction temperature to be set relatively low, acrylic acid selectivity can be increased as a result. Further, by setting the calcination temperature in the calcination step to 460°C or lower, the catalytic active component of the obtained catalyst comes to have the peak intensity ratio I(31.5°)II(22.2°) of 0.20 or less and the peak intensity ratio I(32.6°)/I(22.2°) of 0.40 or less. Since this catalyst has high activity and allows the reaction temperature to be set relatively low, acrylic acid selectivity can be increased as a result.

In the cooling step, the oxygen concentration in the atmosphere is preferably 6 volume% or more, more preferably 8 volume% or more, and even more preferably 10 volume% or more. The upper limit of the oxygen concentration in the atmosphere is preferably 20 volume% or less, more preferably 18 volume% or less.

In the cooling step, the cooling period from the temperature Tc to 100°C is preferably longer than 5 hours, more preferably 5.2 hours or longer, and even more preferably 6 hours or longer. The upper limit of the cooling period from the temperature Tc to 100°C is not particularly limited; however, it is preferably 12 hours or shorter, more preferably 10 hours or shorter, from the viewpoint of enhancing production efficiency of the catalyst.

In the cooling step, it is also preferable to cool from the temperature Tc to 300°C over 3 hours or longer, more preferably 3.5 hours or longer. Thereby, the catalyst of the present invention can be easily obtained, and the obtained catalyst exhibits high acrylic acid selectivity or high acrylic acid yield. The upper limit of the cooling time from the temperature Tc to 300°C is not particularly limited; however, it is preferably 10 hours or shorter, more preferably 8 hours or shorter, and even more preferably 6 hours or shorter, from the viewpoint of enhancing production efficiency of the catalyst.

The cooling period in the cooling step can be easily adjusted by, for example, keeping the inside of the furnace in a windless state, blowing cold air into the furnace, adjusting heating of a heater, introducing a gas with controlled atmosphere in the furnace while controlling its flow rate, or the like. An average cooling rate during cooling from the calcining temperature Tc to 100°C is preferably 0.38°C/min or more, more preferably 0.43°C/min or more, and preferably 1.2°C/min or less, more preferably 1.0°C/min or less. An average cooling rate during cooling from the calcining temperature Tc to 300°C is preferably 0.1°C/min or more, more preferably 0.2°C/min or more, and preferably 1.0°C/min or less, more preferably 0.9°C/min or less.

The calcining temperature in the calcining step and the cooling temperature in the cooling step can be obtained as follows. For example, the dried product, molded product or supported product to be calcined is loaded and placed in a calcining furnace, a temperature sensor is set about the center of a layer of the dried material, molded product or supported product, the calcining step and the cooling step are carried out in this state, and the temperature in each step is measured, whereby the calcining temperature and the cooling temperature can be obtained.

In the first preparing method, the calcined product obtained through the cooling step can be used as the catalyst as it is. In the first preparing method, since the dried product is molded or supported on an inert carrier and then calcined at temperature Tc, the catalyst obtained through the calcining step and the cooling step is formed to be a molded catalyst or a supported catalyst.

In the second preparing method, after the cooling step, a step of molding the calcined product obtained through the cooling step or supporting the calcined product obtained through the cooling step on an inert carrier is conducted (hereinafter referred to as a "second molding/supporting step"). As a result, the catalyst can be obtained as a molded catalyst or a supported catalyst also in the second preparing method. Explanations of the molding method and the supporting method in the second molding/supporting step is referred to the description of the molding method and the supporting method in the first molding/supporting step of the first preparing method.

In the second preparing method, the molded product or the supported product obtained in the second molding/supporting step may be heated at a temperature Tc or lower. The heating temperature (Tₕ) of the heat treatment at this time is not particularly limited as long as it is not higher than the temperature Tc, and is preferably Tc-30°C or lower, and preferably 100°C or higher, more preferably 200°C or higher. A period for the heat treatment may be appropriately adjusted between, for example, 0.5 hours and 12 hours. An atmosphere in the heat treatment is not particularly limited, and the heat treatment can be performed in an inert gas atmosphere such as nitrogen or argon, a reducing gas atmosphere such as hydrogen, or an oxygen-containing gas atmosphere such as air. Among them, the heat treatment is preferably performed in an oxygen-containing gas atmosphere.

In the second preparing method, the heat treatment after the second molding/supporting step is not performed for the purpose of controlling the growth of the crystal structure of the catalytic active component, but for the purpose of volatilizing or decomposing a binder used during molding and supporting as necessary, for example. Since the heat treatment after the second molding/supporting step is not performed for the purpose of controlling the growth of the crystal structure of the catalytic active component, a cooling period and a cooling rate for cooling after the heat treatment are not particularly limited. However, from the viewpoint of production efficiency of the catalyst, in the case of conducting the heat treatment at the temperature Tₕ higher than 100°C, the cooling period from the temperature Tₕ to 100°C is preferably 12 hours or shorter, more preferably 10 hours or shorter, and even more preferably 8 hours or shorter. The cooling rate at this time is preferably 0.45°C/min or more, more preferably 0.55°C/min or more, and even more preferably 0.68°C/min or more. An atmosphere for cooling after the heat treatment is not particularly limited, and can be performed in an inert gas atmosphere such as nitrogen or argon, a reducing gas atmosphere such as hydrogen, or an oxygen-containing gas atmosphere such as air.

The catalyst of the present invention and the catalyst obtained by the preparing method of the present invention are useful as a catalyst for producing acrylic acid from acrolein. A process for producing acrylic acid of the present invention comprises the step of conducting gas-phase catalytic oxidation of acrolein in the presence of the catalyst of the present invention or the catalyst obtained by the method for preparing the catalyst of the present invention to obtain acrylic acid. The process for producing acrylic acid of the present invention may comprise the steps of: obtaining a catalyst by the preparing method of the present invention; and conducting gas-phase catalytic oxidation of acrolein in the presence of the catalyst obtained in the aforesaid step to obtain acrylic acid. According to the process for producing acrylic acid of the present invention, acrylic acid can be produced from acrolein at high yield.

Reaction conditions in the process for producing acrylic acid of the present invention are not particularly limited, and any conditions generally used for this kind of reaction can be adopted. For example, a mixed gas, composed of 1 to 15 volume%, preferably 4 to 12 volume% of acrolein, 0.5 to 25 volume%, preferably 2 to 20 volume% of molecular oxygen, and 0 to 30 volume%, preferably 0 to 25 volume% of steam, and the balance of an inert gas such as nitrogen, may be used as the raw material gas. An acrolein-containing gas obtained by a dehydration reaction of glycerin or a gas-phase catalytic oxidation reaction of propylene, or a mixed gas obtained by adding air or oxygen to the acrolein-containing gas, as necessary, may also be used as the raw material gas. The reaction temperature may be appropriately set within the temperature range of 200°C to 400°C. As heating means, known heating means such as molten salt and an electric heater can be used.

As a reactor, known reactors such as a fixed bed reactor ((multi)tubular reactor), a fluidized bed reactor, and a moving bed reactor can be used, and these reactors in which the catalyst is placed or filled can be used. In the case where a fixed bed reactor is used as the reactor, a space velocity of the raw material gas introduced into the fixed bed reactor is preferably 300 hr⁻¹ or more, more preferably 500 hr⁻¹ or more, even more preferably 1000 hr⁻¹ or more, and preferably 10000 hr⁻¹ or less, more preferably 8000 hr⁻¹ or less, even more preferably 5000 hr⁻¹ or less. The space velocity referred hereto means the amount (0°C, 1 bar standard temperature and pressure (STP) conversion value) of the raw material gas passing through a catalyst layer per unit time.

By the above reaction, an acrylic acid-containing gas is obtained as a reaction product. When the acrylic acid-containing gas is cooled to be condensed or collected with a solvent, a crude acrylic acid-containing liquid is obtained. If necessary, the crude acrylic acid-containing liquid may be purified by known purification means (e.g., distillation, crystallization, stripping, extraction) to obtain highly purified acrylic acid.

This application claims priority to Japanese Patent Application No. 2020-148124 filed on September 3, 2020. All of the contents of the Japanese Patent Applications No. 2020-148124 filed on September 3, 2020, are incorporated by reference herein.

### EXAMPLES

Hereinafter, the present invention is described more specifically by reference to Examples; however, the present invention is not limited to the following Examples, appropriate modifications can be made within the scope that can conform to the gist of the above and below descriptions, and all of them are included in the technical scope of the present invention. In the following description, "parts" means "parts by mass" unless otherwise specified.

### (1) Preparation of catalyst

### (1-1) Preparation Example 1 (Preparation example of catalyst 1 used in Example 1)

1000 parts of pure water was heated while stirring, and 100 parts of ammonium paramolybdate, 22.1 parts of ammonium metavanadate, and 12.8 parts of ammonium paratungstate were dissolved therein. Separately, 100 parts of pure water was heated while stirring, and 11.4 parts of copper nitrate was dissolved therein. The two solutions obtained were mixed, and 4.1 parts of antimony trioxide was added thereto to obtain a raw material mixed solution. This raw material mixed solution was spray-dried, and the resulting dried product was sieved to a size of 250 µm or smaller, thereby obtaining a dried powder. 300 parts of a spherical carrier made of α-alumina having an average particle diameter of 4 mm was put into a centrifugal fluidized coating apparatus, pure water was added thereto as a binder to immerse the carrier, and then the dried powder was supported on the carrier and dried with hot air of about 90°C, thereby obtaining a catalyst precursor. The obtained catalyst precursor was placed in a crucible and put in a box-type calcining furnace. The oxygen concentration in the furnace was adjusted to 10 volume%, the temperature was raised from room temperature at a rate of 2°C/min, and then calcining was carried out at 380°C for 6.0 hours. After that, the oxygen concentration in the furnace was adjusted to 18 volume%, cooling from 380°C to 300°C was carried out taking for 3.0 hours, and cooling from 380°C to 100°C was carried out taking for 5.3 hours, thereby obtaining a catalyst 1. The supporting rate of the catalyst 1 was 30 mass%, and the metal element composition excluding oxygen was Mo₁₂V_{4.0}W_{1.0}Cu_{1.0}Sb_{0.6}. The supporting rate was obtained from the following formula: supporting rate (mass%) = (mass (g) of catalyst powder supported)/(mass (g) of carrier used) × 100.

### (1-2) Preparation Example 2 (Preparation example of catalyst 2 used in Example 2)

A catalyst precursor was prepared in the same manner as in Preparation Example 1, except that 100 parts of ammonium paramolybdate, 24.9 parts of ammonium metavanadate, 17.9 parts of ammonium paratungstate, 17.1 parts of copper nitrate, and 3.4 parts of antimony trioxide were used as raw materials in Preparation Example 1. The obtained catalyst precursor was placed in a crucible, the oxygen concentration in the furnace was adjusted to 10 volume% in the box-type calcining furnace, the temperature was raised from room temperature at a rate of 2°C/min, and then calcining was carried out at 400°C for 6.0 hours. After that, the oxygen concentration in the furnace was adjusted to 14 volume%, cooling from 400°C to 300°C was carried out taking for 3.5 hours, and cooling from 400°C to 100°C was carried out taking for 6.0 hours, thereby obtaining a catalyst 2. The supporting rate of the catalyst 2 was 30 mass%, and the metal element composition excluding oxygen was Mo₁₂V_{4.5}Cu_{1.5}W_{1.4}Sb_{0.5}.

### (1-3) Preparation Example 3 (Preparation example of catalyst 3 used in Example 3)

A catalyst precursor was prepared in the same manner as in Preparation Example 1, except that 100 parts of ammonium paramolybdate, 22.1 parts of ammonium metavanadate, 15.3 parts of ammonium paratungstate, 6.8 parts of copper nitrate, and 4.8 parts of antimony trioxide were used as raw materials in Preparation Example 1. The obtained catalyst precursor was placed in a crucible, the oxygen concentration in the furnace was adjusted to 10 volume% in the box-type calcining furnace, the temperature was raised from room temperature at a rate of 2°C/min, and then calcining was carried out at 410°C for 6.0 hours. After that, the oxygen concentration in the furnace was adjusted to 10 volume%, cooling from 410°C to 300°C was carried out taking for 4.0 hours, and cooling from 410°C to 100°C was carried out taking for 6.5 hours, thereby obtaining a catalyst 3. The supporting rate of the catalyst 3 was 30 mass%, and the metal element composition excluding oxygen was Mo₁₂V_{4.0}Cu_{0.6}W_{1.2}Sb_{0.7}.

### (1-4) Preparation Example 4 (Preparation example of catalyst 4 used in Example 4)

A catalyst precursor was prepared in the same manner as in Preparation Example 1, except that 100 parts of ammonium paramolybdate, 22.1 parts of ammonium metavanadate, 12.8 parts of ammonium paratungstate, 14.8 parts of copper nitrate, and 3.4 parts of antimony trioxide were used as raw materials in Preparation Example 1. The obtained catalyst precursor was placed in a crucible, the oxygen concentration in the furnace was adjusted to 10 volume% in the box-type calcining furnace, the temperature was raised from room temperature at a rate of 2°C/min, and then calcining was carried out at 400°C for 6.0 hours. After that, the oxygen concentration in the furnace was adjusted to 10 volume%, cooling from 400°C to 300°C was carried out taking for 3.5 hours, and cooling from 400°C to 100°C was carried out taking for 6.0 hours, thereby obtaining a catalyst 4. The supporting rate of the catalyst 4 was 30 mass%, and the metal element composition excluding oxygen was Mo₁₂V_{4.0}Cu_{1.3}W_{1.0}Sb_{0.5}.

### (1-5) Preparation Example 5 (Preparation example of catalyst 5 used in Example 5)

A catalyst precursor was prepared in the same manner as in Preparation Example 1, except that 100 parts of ammonium paramolybdate, 24.9 parts of ammonium metavanadate, 15.3 parts of ammonium paratungstate, 18.3 parts of copper nitrate, and 4.1 parts of antimony trioxide were used as raw materials in Preparation Example 1. The obtained catalyst precursor was placed in a crucible, the oxygen concentration in the furnace was adjusted to 10 volume% in the box-type calcining furnace, the temperature was raised from room temperature at a rate of 2°C/min, and then calcining was carried out at 420°C for 6.0 hours. After that, the oxygen concentration in the furnace was adjusted to 12 volume%, cooling from 420°C to 300°C was carried out taking for 4.5 hours, and cooling from 420°C to 100°C was carried out taking for 7.0 hours, thereby obtaining a catalyst 5. The supporting rate of the catalyst 5 was 30 mass%, and the metal element composition excluding oxygen was Mo₁₂V_{4.5}Cu_{1.6}W_{1.2}Sb_{0.6}.

### (1-6) Preparation Example 6 (Preparation example of catalyst 6 used in Example 6)

A catalyst precursor was prepared in the same manner as in Preparation Example 1, except that 100 parts of ammonium paramolybdate, 22.1 parts of ammonium metavanadate, 12.8 parts of ammonium paratungstate, 20.5 parts of copper nitrate, and 3.4 parts of antimony trioxide were used as raw materials in Preparation Example 1. The obtained catalyst precursor was placed in a crucible, the oxygen concentration in the furnace was adjusted to 10 volume% in the box-type calcining furnace, the temperature was raised from room temperature at a rate of 2°C/min, and then calcining was carried out at 430°C for 6.0 hours. After that, the oxygen concentration in the furnace was adjusted to 12 volume%, cooling from 430°C to 300°C was carried out taking for 5.0 hours, and cooling from 430°C to 100°C was carried out taking for 7.5 hours, thereby obtaining a catalyst 6. The supporting rate of the catalyst 6 was 30 mass%, and the metal element composition excluding oxygen was Mo₁₂V_{4.0}Cu_{1.8}W_{1.0}Sb_{0.5}.

### (1-7) Preparation Example 7 (Preparation example of catalyst 7 used in Example 7)

A catalyst precursor was prepared in the same manner as in Preparation Example 1, except that 100 parts of ammonium paramolybdate, 19.3 parts of ammonium metavanadate, 12.8 parts of ammonium paratungstate, 34.2 parts of copper nitrate, and 3.4 parts of antimony trioxide were used as raw materials in Preparation Example 1. The obtained catalyst precursor was placed in a crucible, the oxygen concentration in the furnace was adjusted to 10 volume% in the box-type calcining furnace, the temperature was raised from room temperature at a rate of 2°C/min, and then calcining was carried out at 420°C for 6.0 hours. After that, the oxygen concentration in the furnace was adjusted to 21 volume%, cooling from 420°C to 300°C was carried out taking for 4.5 hours, and cooling from 420°C to 100°C was carried out taking for 7.0 hours, thereby obtaining a catalyst 7. The supporting rate of the catalyst 7 was 30 mass%, and the metal element composition excluding oxygen was Mo₁₂V_{3.5}Cu_{3.0}W_{1.0}Sb_{0.5}.

### (1-8) Preparation Example 8 (Preparation example of catalyst 8 used in Example 8)

A catalyst precursor was prepared in the same manner as in Preparation Example 1, except that 100 parts of ammonium paramolybdate, 33.1 parts of ammonium metavanadate, 12.8 parts of ammonium paratungstate, 22.8 parts of copper nitrate, and 2.8 parts of antimony trioxide were used as raw materials in Preparation Example 1. The obtained catalyst precursor was placed in a crucible, the oxygen concentration in the furnace was adjusted to 10 volume% in the box-type calcining furnace, the temperature was raised from room temperature at a rate of 2°C/min, and then calcining was carried out at 400°C for 6.0 hours. After that, the oxygen concentration in the furnace was adjusted to 10 volume%, cooling from 400°C to 300°C was carried out taking for 3.5 hours, and cooling from 400°C to 100°C was carried out taking for 6.0 hours, thereby obtaining a catalyst 8. The supporting rate of the catalyst 8 was 30 mass%, and the metal element composition excluding oxygen was Mo₁₂V_{6.0}Cu_{2.0}W_{1.0}Sb_{0.4}.

### (1-9) Preparation Example 9 (Preparation example of catalyst 9 used in Example 9)

A catalyst precursor was prepared in the same manner as in Preparation Example 1, except that 100 parts of ammonium paramolybdate, 16.6 parts of ammonium metavanadate, 10.2 parts of ammonium paratungstate, 3.4 parts of copper nitrate, and 2.1 parts of antimony trioxide were used as raw materials in Preparation Example 1. The obtained catalyst precursor was placed in a crucible, the oxygen concentration in the furnace was adjusted to 10 volume% in a box-type calcining furnace, the temperature was raised from room temperature at a rate of 2°C/min, and then calcining was carried out at 440°C for 6.0 hours. After that, the oxygen concentration in the furnace was adjusted to 20 volume%, cooling from 440°C to 300°C was carried out taking for 6.0 hours, and cooling from 440°C to 100°C was carried out taking for 8.5 hours, thereby obtaining a catalyst 9. The supporting rate of the catalyst 9 was 30 mass%, and the metal element composition excluding oxygen was Mo₁₂V_{3.0}Cu_{0.3}W_{0.8}Sb_{0.3}.

### (1-10) Preparation Example 10 (Preparation example of catalyst 10 used in Example 10)

A catalyst precursor was prepared in the same manner as in Preparation Example 3. The obtained catalyst precursor was placed in a crucible, the oxygen concentration in the furnace was adjusted to 1 volume% in the box-type calcining furnace, the temperature was raised from room temperature at a rate of 2°C/min, and then calcining was carried out at 400°C for 6.0 hours. After that, the oxygen concentration in the furnace was adjusted to 10 volume%, cooling from 400°C to 300°C was carried out taking for 3.5 hours, and cooling from 400°C to 100°C was carried out taking for 6.0 hours, thereby obtaining a catalyst 10. The supporting rate of the catalyst 10 was 30 mass%, and the metal element composition excluding oxygen was Mo₁₂V_{4.0}Cu_{0.6}W_{1.2}Sb_{0.7}.

### (1-11) Preparation Example 11 (Preparation example of catalyst 11 used in Example 11)

A catalyst precursor was prepared in the same manner as in Preparation Example 8. The obtained catalyst precursor was placed in a crucible, the oxygen concentration in the furnace was adjusted to 21 volume% in the box-type calcining furnace, the temperature was raised from room temperature at a rate of 2°C/min, and then calcining was carried out at 400°C for 6.0 hours. After that, the oxygen concentration in the furnace was adjusted to 10 volume%, cooling from 400°C to 300°C was carried out taking for 3.5 hours, and cooling from 400°C to 100°C was carried out taking for 6.0 hours, thereby obtaining a catalyst 11. The supporting rate of the catalyst 11 was 30 mass%, and the metal element composition excluding oxygen was Mo₁₂V_{6.0}Cu_{2.0}W_{1.0}Sb_{0.4}.

### (1-12) Preparation Example 12 (Preparation example of catalyst 12 used in Example 12)

A catalyst precursor was prepared in the same manner as in Preparation Example 6. The obtained catalyst precursor was placed in a crucible, and the oxygen concentration in the furnace was adjusted to 10 volume% in the box-type calcining furnace, the temperature was raised from room temperature at a rate of 2°C/min, and then calcining was carried out at 420°C for 1.0 hour. After that, the oxygen concentration in the furnace was adjusted to 6 volume%, cooling from 420°C to 300°C was carried out taking for 4.5 hours, and cooling from 420°C to 100°C was carried out taking for 7.0 hours, thereby obtaining a catalyst 12. The supporting rate of the catalyst 12 was 30 mass%, and the metal element composition excluding oxygen was Mo₁₂V_{4.0}Cu_{1.8}W_{1.0}Sb_{0.5}.

### (1-13) Preparation Example 13 (Preparation example of catalyst 13 used in Example 13)

1000 parts of pure water was heated while stirring, and 100 parts of ammonium paramolybdate, 33.1 parts of ammonium metavanadate, and 12.8 parts of ammonium paratungstate were dissolved therein. Separately, 100 parts of pure water was heated with stirring, and 22.8 parts of copper nitrate was dissolved therein. A catalyst precursor was prepared in the same manner as in Preparation Example 1, except that the two solutions obtained were mixed, and 8.2 parts of cobalt nitrate and 2.8 parts of antimony trioxide were added thereto to obtain a raw material mixed solution. The obtained catalyst precursor was placed in a crucible, the oxygen concentration in the furnace was adjusted to 10 volume% in the box-type calcining furnace, the temperature was raised from room temperature at a rate of 2°C/min, and then calcining was carried out at 390°C for 6.0 hours. After that, the oxygen concentration in the furnace was adjusted to 10 volume%, cooling from 390°C to 300°C was carried out taking for 3.5 hours, and cooling from 390°C to 100°C was carried out taking for 6.0 hours, thereby obtaining a catalyst 13. The supporting rate of the catalyst 13 was 30 mass%, and the metal element composition excluding oxygen was Mo₁₂V_{6.0}Cu_{2.0}W_{1.0}Sb_{0.4}Co_{0.6}.

### (1-14) Preparation Example 14 (Preparation example of catalyst 14 used in Example 14)

A catalyst precursor was prepared in the same manner as in Preparation Example 13, except that 0.4 parts of magnesium oxide was used instead of 8.2 parts of cobalt nitrate. The obtained catalyst precursor was placed in a crucible, the oxygen concentration in the furnace was adjusted to 10 volume% in the box-type calcining furnace, the temperature was raised from room temperature at a rate of 2°C/min, and then calcining was carried out at 400°C for 6.0 hours. After that, the oxygen concentration in the furnace was adjusted to 10 volume%, cooling from 400°C to 300°C was carried out taking for 3.5 hours, and cooling from 400°C to 100°C was carried out taking for 6.0 hours, thereby obtaining a catalyst 14. The supporting rate of the catalyst 14 was 30 mass%, and the metal element composition excluding oxygen was Mo₁₂V_{6.0}Cu_{2.0}W_{1.0}Sb_{0.4}Mg_{0.2}.

### (1-15) Preparation Example 15 (Preparation example of catalyst 15 used in Example 15)

1000 parts of pure water was heated while stirring, and 100 parts of ammonium paramolybdate, 22.1 parts of ammonium metavanadate, and 12.8 parts of ammonium paratungstate were dissolved therein. Separately, 100 parts of pure water was heated with stirring, and 20.5 parts of copper nitrate was dissolved therein. A catalyst precursor was prepared in the same manner as in Preparation Example 1, except that the two solutions obtained were mixed, and 6.3 parts of aluminum oxide and 3.4 parts of antimony trioxide were added thereto to obtain a raw material mixed solution. The obtained catalyst precursor was placed in a crucible, the oxygen concentration in the furnace was adjusted to 10 volume% in the box-type calcining furnace, the temperature was raised from room temperature at a rate of 2°C/min, and then calcining was carried out at 400°C for 6.0 hours. After that, the oxygen concentration in the furnace was adjusted to 10 volume%, cooling from 400°C to 300°C was carried out taking for 3.5 hours, and cooling from 400°C to 100°C was carried out taking for 6.0 hours, thereby obtaining a catalyst 15. The supporting rate of the catalyst 15 was 30 mass%, and the metal element composition excluding oxygen was Mo₁₂V_{4.0}Cu_{1.8}W_{1.0}Sb_{0.5}Al_{1.3}.

### (1-16) Preparation Example 16 (Preparation example of catalyst 16 used in Example 16)

A dried powder was prepared in the same manner as in Preparation Example 1, except that 100 parts of ammonium paramolybdate, 33.1 parts of ammonium metavanadate, 12.8 parts of ammonium paratungstate, 22.8 parts of copper nitrate, and 2.8 parts of antimony trioxide were used as raw materials in Preparation Example 1. The obtained dried powder was placed in a crucible, the oxygen concentration in the furnace was adjusted to 10 volume% in the box-type calcining furnace, the temperature was raised from room temperature at a rate of 2°C/min, and then the powder was calcined at 400°C for 6.0 hours. After that, the oxygen concentration in the furnace was adjusted to 10 volume%, cooling from 400°C to 300°C was carried out taking for 3.5 hours, and cooling from 400°C to 100°C was carried out taking for 6.0 hours, thereby obtaining a calcined product. The obtained calcined product was pulverized using a ball mill to obtain a calcined powder. 300 parts of a spherical carrier made of α-alumina having an average particle diameter of 4 mm was put into a centrifugal fluidized coating apparatus, 1 mass% hydroxyethyl cellulose solution was added thereto as a binder to immerse the carrier, and then the calcined powder was supported on the carrier and dried with hot air of about 90°C, thereby obtaining a supported product. The obtained supported product was placed in a crucible, the oxygen concentration in the furnace was adjusted to 10 volume% in the box-type calcining furnace, the temperature was raised from room temperature at a rate of 2°C/min, then calcining was carried out at 300°C for 2.0 hours, and cooling from 300°C to 100°C was carried out taking for 2.5 hours, thereby obtaining a catalyst 16. The supporting rate of the catalyst 16 was 30 mass%, and the metal element composition excluding oxygen was Mo₁₂V_{6.0}Cu_{2.0}W_{1.0}Sb_{0.4}.

### (1-17) Preparation Example 17 (Preparation example of catalyst 17 used in Comparative Example 1)

A catalyst precursor was prepared in the same manner as in Preparation Example 9. The obtained catalyst precursor was placed in a crucible, the oxygen concentration in the furnace was adjusted to 10 volume% in the box-type calcining furnace, the temperature was raised from room temperature at a rate of 2°C/min, and then calcining was carried out at 440°C for 6.0 hours. After that, the oxygen concentration in the furnace was adjusted to 21 volume%, cooling from 440°C to 300°C was carried out taking for 2.0 hours, and cooling from 440°C to 100°C was carried out taking for 4.0 hours, thereby obtaining a catalyst 17. The supporting rate of the catalyst 17 was 30 mass%, and the metal element composition excluding oxygen was Mo₁₂V_{3.0}Cu_{0.3}W_{0.8}Sb_{0.3}.

### (1-18) Preparation Example 18 (Preparation example of catalyst 18 used in Comparative Example 2)

A catalyst precursor was prepared in the same manner as in Preparation Example 9. The obtained catalyst precursor was placed in a crucible, and the oxygen concentration in the furnace was adjusted to 10 volume% in the box-type calcining furnace, the temperature was raised from room temperature at a rate of 2°C/min, and then calcining was carried out at 440°C for 6.0 hours. After that, the oxygen concentration in the furnace was adjusted to 25 volume%, cooling from 440°C to 300°C was carried out taking for 6.0 hours, and cooling from 440°C to 100°C was carried out taking for 8.5 hours, thereby obtaining a catalyst 18. The supporting rate of the catalyst 18 was 30 mass%, and the metal element composition excluding oxygen was Mo₁₂V_{3.0}Cu_{0.3}W_{0.8}Sb_{0.3}.

### (1-19) Preparation Example 19 (Preparation example of catalyst 19 used in Comparative Example 3)

A catalyst precursor was prepared in the same manner as in Preparation Example 9. The obtained catalyst precursor was placed in a crucible, the oxygen concentration in the furnace was adjusted to 10 volume% in the box-type calcining furnace, the temperature was raised from room temperature at a rate of 2°C/min, and then calcining was carried out at 440°C for 6.0 hours. After that, the oxygen concentration in the furnace was adjusted to 30 volume%, cooling from 440°C to 300°C was carried out taking for 6.0 hours, and cooling from 440°C to 100°C was carried out taking for 8.5 hours, thereby obtaining a catalyst 19. The supporting rate of the catalyst 19 was 30 mass%, and the metal element composition excluding oxygen was Mo₁₂V_{3.0}Cu_{0.3}W_{0.8}Sb_{0.3}.

### (1-20) Preparation Example 20 (Preparation example of catalyst 20 used in Comparative Example 4)

A catalyst precursor was prepared in the same manner as in Preparation Example 8. The obtained catalyst precursor was placed in a crucible, the oxygen concentration in the furnace was adjusted to 10 volume% in the box-type calcining furnace, the temperature was raised from room temperature at a rate of 2°C/min, and then calcining was carried out at 420°C for 6.0 hours. After that, the oxygen concentration in the furnace was adjusted to 5 volume%, cooling from 420°C to 300°C was carried out taking for 4.5 hours, and cooling from 420°C to 100°C was carried out taking for 7.0 hours, thereby obtaining a catalyst 20. The supporting rate of the catalyst 20 was 30 mass%, and the metal element composition excluding oxygen was Mo₁₂V_{6.0}Cu_{2.0}W_{1.0}Sb_{0.4}.

### (1-21) Preparation Example 21 (Preparation example of catalyst 21 used in Comparative Example 5)

A catalyst precursor was prepared in the same manner as in Preparation Example 8. The obtained catalyst precursor was placed in a crucible, the oxygen concentration in the furnace was adjusted to 10 volume% in the box-type calcining furnace, the temperature was raised from room temperature at a rate of 2°C/min, and then calcining was carried out at 480°C for 6.0 hours. After that, the oxygen concentration in the furnace was adjusted to 10 volume%, cooling from 480°C to 300°C was carried out taking for 7.0 hours, and cooling from 480°C to 100°C was carried out taking for 9.5 hours, thereby obtaining a catalyst 21. The supporting rate of the catalyst 21 was 30 mass%, and the metal element composition excluding oxygen was Mo₁₂V_{6.0}Cu_{2.0}W_{1.0}Sb_{0.4}.

### (1-22) Preparation Example 22 (Preparation example of catalyst 22 used in Comparative Example 6)

A catalyst precursor was prepared in the same manner as in Preparation Example 8. The obtained catalyst precursor was placed in a crucible, the oxygen concentration in the furnace was adjusted to 10 volume% in the box-type calcining furnace, the temperature was raised from room temperature at a rate of 2°C/min, and then calcining was carried out at 370°C for 6.0 hours. After that, the oxygen concentration in the furnace was adjusted to 10 volume%, and cooling from 370°C to 300°C was carried out taking for 3.0 hours, and cooling from 370°C to 100°C was carried out taking for 5.0 hours, thereby obtaining a catalyst 22. The supporting rate of the catalyst 22 was 30 mass%, and the metal element composition excluding oxygen was Mo₁₂V_{6.0}Cu_{2.0}W_{1.0}Sb_{0.4}.

Preparation conditions of the catalyst and catalyst composition in each of the above preparation examples are shown in Tables 1 and 2.

### (2) X-ray diffraction analysis of catalytic active component

The catalyst obtained in each preparation example was shaken in a closed container to peel off the catalytic active component supported on a surface of the carrier, and then sieved to obtain powder with a size of 150 µm or less. X-ray diffraction analysis of the obtained powder with Cu-Kα radiation (X-ray output: 40 mA-45 kV, Kα1 ray wavelength: 1.5406 Å) was carried out using an X-ray diffractometer (PHILIPS X'pertPRO). The results of X-ray diffraction analysis of the catalytic active component in each catalyst is shown in Tables 3 and 4.

### (3) Production of acrylic acid from acrolein

A U-shaped reaction tube, made of SUS, with a total length of 300 mm and an inner diameter of 18 mm was filled with the catalyst so that a layer length was 100 mm. A mixed gas consisting of 2 volume% of acrolein, 3 volume% of oxygen, 10 volume% of steam and 85 volume% of nitrogen was introduced thereinto at a space velocity of 5000 hr⁻¹ (STP) to proceed an acrolein oxidation reaction. The reaction temperature was adjusted so that the conversion of acrolein was around 93.5%. The results of performance evaluation of each catalyst are shown in Tables 3 and 4.

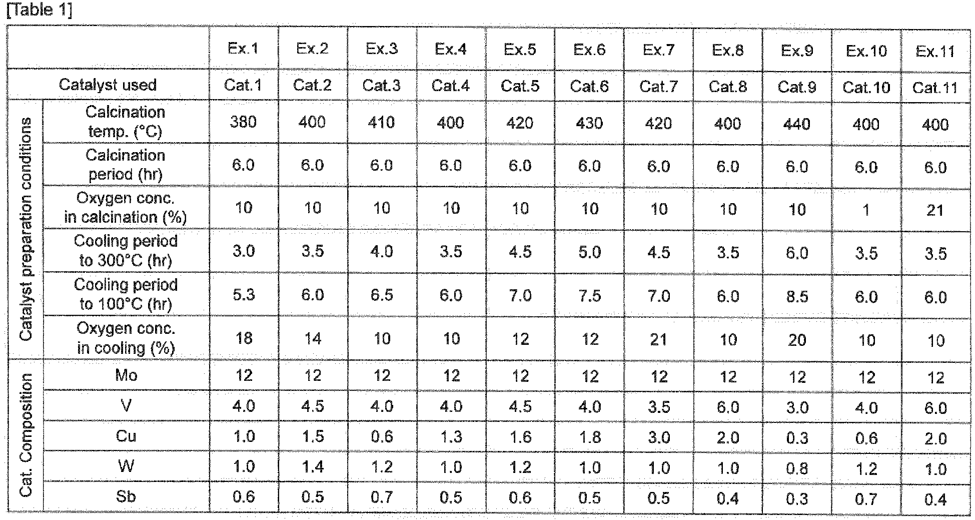

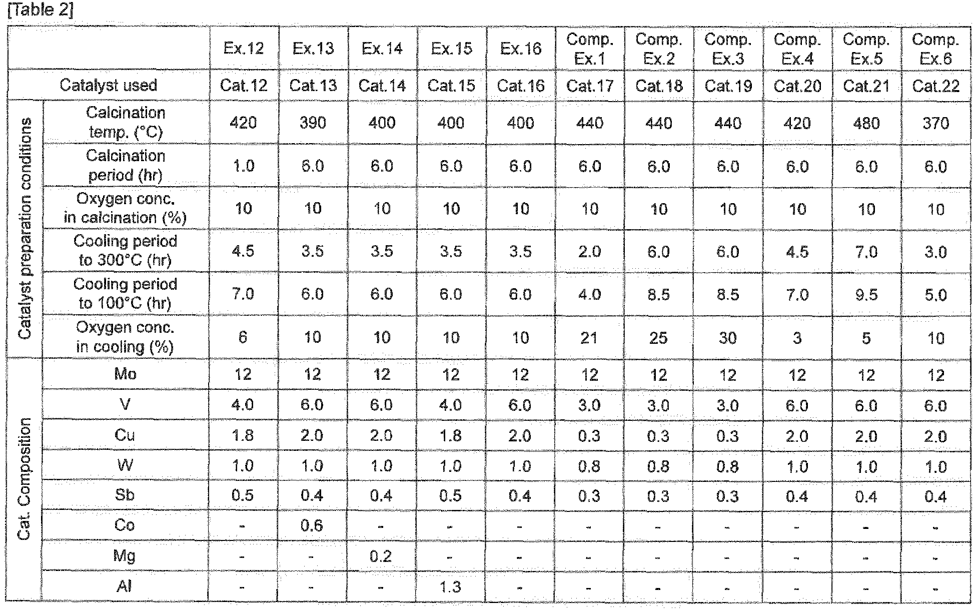

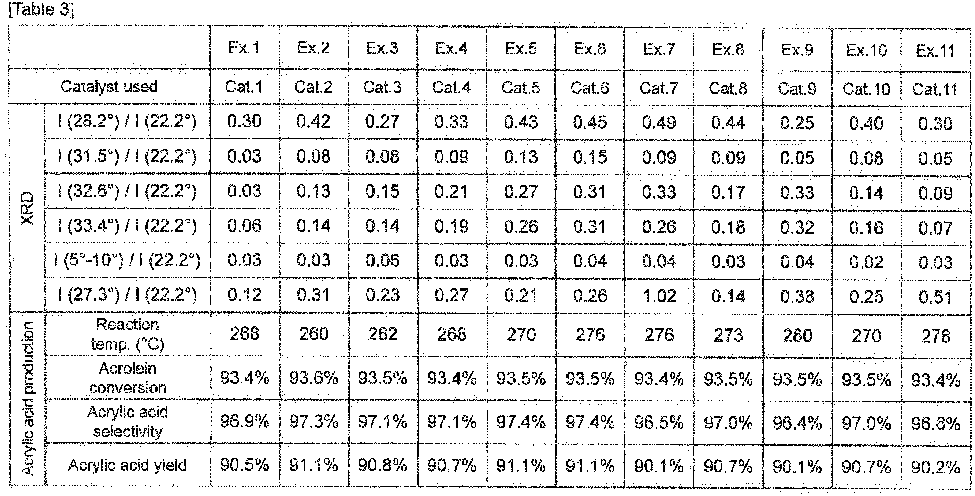

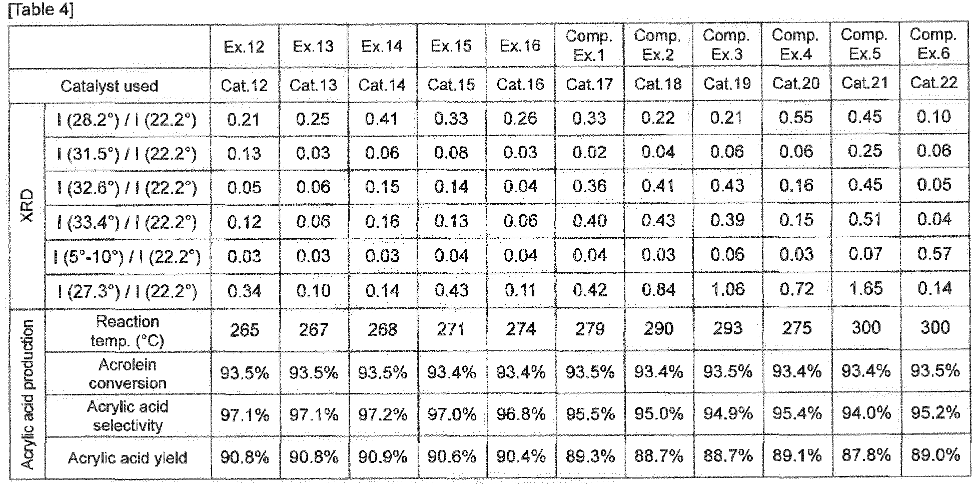

In the catalysts 1 to 16 used in Examples, the catalytic active components had diffraction peaks at 2θ = 22.2° ± 0.3°, 28.2° ± 0.3°, 31.5° ± 0.3° and 32.6° ± 0.3° in the X-ray diffraction analysis using Cu-Kα radiation, and the peak intensity ratio I(28.2°)/I(22.2°) was 0.20 or more and 0.50 or less, the peak intensity ratio I(31.5°)/I(22.2°) was 0.03 or more and 0.20 or less, and the peak intensity ratio I(32.6°)/I(22.2°) was 0.03 or more and 0.40 or less. Meanwhile, in the catalysts 17 to 22 used in Comparative Examples, the catalytic active components had diffraction peaks at 2θ = 22.2° ± 0.3°, 28.2° ± 0.3, 31.5° ± 0.3° and 32.6° ± 0.3° in X-ray diffraction analysis using Cu-Kα radiation; however, the peak intensity ratio I(31.5°)II(22.2°) was less than 0.03 in the catalyst 17, the peak intensity ratio I(32.6°)/I(22.2°) was more than 0.40 in the catalyst 18 and the catalyst 19, the peak intensity ratio I(28.2°)/I(22.2°) was more than 0.50 in the catalyst 20, the peak intensity ratio I(31.5°)/I(22.2°) was more than 0.20 and the peak intensity ratio I(32.6°)/I(22.2°) was more than 0.40 in the catalyst 21, and the peak intensity ratio I(28.2°)/I(22.2°) was less than 0.20 in the catalyst 22. As a result, when acrylic acid was produced by a gas-phase catalytic oxidation reaction of acrolein using each catalyst, Examples 1 to 16 exhibited higher acrylic acid selectivity and higher acrylic acid yield than Comparative Examples 1 to 6.

The catalysts 1 to 16 used in Examples were prepared by drying the raw material mixed solution of the catalytic active component, calcining at a temperature Tc of 380°C or higher and 460°C or lower, and then cooling from the temperature Tc to 100°C over 5 hours or longer in the atmosphere with the oxygen concentration more than 5 volume% and 21 volume% or less. In addition, cooling from the temperature Tc to 300°C was carried out taking 3 hours or longer. As a result, the catalysts exhibiting high acrylic acid selectivity and high acrylic acid yield were obtained, as explained above. Meanwhile, for preparation of the catalyst 17 used in Comparative Example, the cooling period from the temperature Tc to 100°C was shorter than 5 hours, and the cooling period from temperature Tc to 300°C was shorter than 3 hours, in the cooling step following calcination. For preparation of the catalysts 18 to 20, the cooling step following calcination was carried out under the conditions of oxygen concentration of less than 5 volume% or more than 21 volume%. For preparation of the catalyst 21, the temperature Tc in the calcining step was higher than 460°C. For preparation of the catalyst 22, the temperature Tc in the calcining step was lower than 380°C. As a result, the obtained catalysts exhibited lower acrylic acid selectivity and acrylic acid yield than those of Examples, as explained above.

### INDUSTRY APPLICABILITY

The catalyst of the present invention can produce acrylic acid from acrolein. The obtained acrylic acid can be used as a raw material for various acrylic acid derivative products such as acrylic acid esters, sodium polyacrylate and acrylic resins.

## Claims

1. A catalyst for producing acrylic acid from acrolein, comprising a catalytic active component containing molybdenum, vanadium and copper, wherein:
the catalytic active component has diffraction peaks at 2θ = 22.2° ± 0.3°, 28.2° ± 0.3°, 31.5° ± 0.3°, and 32.6° ± 0.3° in X-ray diffraction analysis using Cu-Kα radiation;
a ratio I(28.2°)/I(22.2°) of peak intensity at 2θ = 28.2° ± 0.3° and peak intensity at 2θ = 22.2° ± 0.3° is 0.20 or more and 0.50 or less;
a ratio I(31.5°)/I(22.2°) of peak intensity at 2θ = 31.5° ± 0.3° and the peak intensity at 2θ = 22.2° ± 0.3° is 0.03 or more and 0.20 or less; and
a ratio I(32.6°)/I(22.2°) of peak intensity at 2θ = 32.6° ± 0.3° and the peak intensity at 2θ = 22.2° ± 0.3° is 0.03 or more and 0.40 or less.

2. The catalyst for producing acrylic acid according to claim 1, wherein:
the catalytic active component further has a diffraction peak at 2θ = 33.4° ± 0.3° in the X-ray diffraction analysis;
a ratio I(33.4°)/I(22.2°) of peak intensity at 2θ = 33.4° ± 0.3° and the peak intensity at 2θ = 22.2° ± 0.3° is 0.06 or more and 0.40 or less; and
a ratio I(5°-10°)/I(22.2°) of the highest intensity in a range of 2θ = 5° to 10° and the peak intensity at 2θ = 22.2° ± 0.3° is 0.06 or less.

3. The catalyst for producing acrylic acid according to claim 1 or 2, wherein:
the catalytic active component further has a diffraction peak at 2θ = 27.3° ± 0.3° in the X-ray diffraction analysis; and
a ratio I(27.3°)/I(22.2°) of peak intensity at 2θ = 27.3° ± 0.3° and the peak intensity at 2θ = 22.2° ± 0.3° is 0.10 or more and 0.60 or less.

4. The catalyst for producing acrylic acid according to any one of claims 1 to 3, wherein:
the catalytic active component has a composition represented by the following formula (1):
MoₐV_{b}Cu_{c}A_{d}BₑC_{f}D_{g}EₕOᵢ ... (1),
wherein Mo is molybdenum, V is vanadium, Cu is copper, A represents at least one element selected from the group consisting of tungsten, niobium and tantalum, B represents at least one element selected from the group consisting of antimony and tin, C represents at least one element selected from the group consisting of chromium, manganese, iron, cobalt, nickel, zinc, zirconium, tellurium, cerium and bismuth, D represents at least one element selected from the group consisting of alkali metals and alkaline earth metals, E represents at least one element selected from the group consisting of silicon, aluminum and titanium, O is oxygen, a to i mean atomic ratios of Mo, V, Cu, A, B, C, D, E and O, respectively, and meet inequalities: a=12, 2≤b≤14, 0<c≤5, 0<d≤10, 0<e≤5, 0≤f≤5, 0≤g≤3, 0≤h≤30, respectively, and i is a numerical value determined by oxidation states of respective elements.

5. A method for preparing a catalyst for producing acrylic acid from acrolein, comprising the steps of:
obtaining a raw material mixed solution containing molybdenum, vanadium and copper;
drying the raw material mixed solution to obtain a dried product;
molding the dried product or supporting the dried product on an inert carrier to obtain a molded product or a supported product;
calcining the molded product or the supported product at a temperature Tc of 380°C or higher and 460°C or lower; and
cooling from the temperature Tc to 100°C over 5 hours or longer, after the calcining, in an atmosphere with an oxygen concentration of more than 5 volume% and 21 volume% or less.

6. A method for preparing a catalyst for producing acrylic acid from acrolein, comprising the steps of:
obtaining a raw material mixed solution containing molybdenum, vanadium and copper;
drying the raw material mixed solution to obtain a dried product;
calcining the dried product at a temperature Tc of 380°C or higher and 460°C or lower;
cooling from the temperature Tc to 100°C over 5 hours or longer, after the calcining, in an atmosphere with an oxygen concentration of more than 5 volume% and 21 volume% or less; and
molding a calcined product obtained through the cooling step or supporting a calcined product obtained through the cooling step on an inert carrier.

7. The method for preparing a catalyst for producing acrylic acid according to claim 5 or 6, wherein
cooling from the temperature Tc to 300°C over 3 hours or longer in the cooling step.

8. A process for producing acrylic acid comprising the step of conducing gas-phase catalytic oxidation of acrolein in the presence of the catalyst according to any one of claims 1 to 4 or the catalyst obtained by the preparing method according to any one of claims 5 to 7.
